**Europäisches Patentamt**

(19) **European Patent Office**

**Office européen des brevets**

(11) Publication number : **0 341 830 B1**

(12) **EUROPEAN PATENT SPECIFICATION**

(45) Date of publication of patent specification :
17.06.92 Bulletin 92/25

(51) Int. Cl.⁵: **A61M 25/00**

(21) Application number : **89303577.4**

(22) Date of filing : **11.04.89**

(54) Splittable introducer catheter with modified tip.

(30) Priority : **09.05.88 US 191523**

(43) Date of publication of application :
**15.11.89 Bulletin 89/46**

(45) Publication of the grant of the patent :
**17.06.92 Bulletin 92/25**

(84) Designated Contracting States :
**AT BE CH DE ES FR GB GR IT LI LU NL SE**

(56) References cited :
**EP-A- 0 279 015**
**US-A- 4 412 832**
**US-A- 4 661 300**

(73) Proprietor : **Becton Dickinson and Company**
**One Becton Drive**
**Franklin Lakes New Jersey 07417-1880 (US)**

(72) Inventor : **Pearsall, Harold I**
**2609 Walford Drive**
**Centerville Ohio (US)**

(74) Representative : **Ruffles, Graham Keith et al**
**MARKS & CLERK 57-60 Lincoln's Inn Fields**
**London WC2A 3LS (GB)**

## Description

BACKGROUND AND STATEMENT OF THE INVENTION

The invention described in this application is related to the subject matter in US-A- 4,661,300, on which the preamble of Claim 1 is based and in EP-A-0 279 015, which is not comprised in the state of the art as defined in Article 54(3).

This invention relates generally to splittable introducer catheters for use in inserting long-term catheter arrangements into patients and to a method for producing such catheters. That is, when attempting to insert long-line catheters into the human body the procedure includes making the venipuncture with a hollow needle that carries coaxially thereover a splittable placement or introducer catheter of the kind to which this invention is directed. Once the vein has been penetrated, and blood is visible in the flashback chamber of such an assembly, the needle and flashback chamber are removed leaving the placement catheter through the site of the puncture and into the vein.

Thereafter, a long-line catheter or guidewire is threaded through the placed catheter, and thereafter the catheter is removed from the vein by axial sliding movement along the guidewire, for example, leaving the guidewire or long-line catheter in place. The removed placement or introducer catheter is designed to split longitudinally from its coaxial position over the guidewire or long-line catheter, so that it may be readily removed without any difficulty or irritation to the patient.

With respect to the above-noted EP-A-0 279 015, that application teaches an invention for producing a splittable introducer or placement catheter with diametrically opposed longitudinally extending grooves positioned in the outer surface of the placement catheter. These grooves facilitate the stripping of the introducer catheter once the guidewire and long-line catheter are in place. However, the single-most difficult part of catheters of the kind discussed herein is the entry when carried over a needle and through the skin of a patient and any trauma or other undesirable circumstances caused thereby. While the longitudinally positioned grooves of the catheter discussed above facilitate to a great extent the splitting of the placement catheter for ultimate removal, the grooves are not only in the main body of the catheter, but also in the tip portion thereof. The grooves in the tip may cause a greater trauma than just the needle upon insertion through the skin of a patient.

Moreover, because of the property of an easily longitudinally split body, sometimes during the initial insertion through the skin of a patient, the placement catheter tip may peel back, which is a problem with over-the-needle placement catheters of this kind.

With this invention, by contrast, a catheter with longitudinal grooves of the kind taught in the above-captioned EP-A-0 279 015 is modified at the tip in order to provide a tapered tip similar to the one taught and claimed in the above-noted US-A-4,661,300. Because of this, the tip has no grooves, but rather, the grooves end at the point where the taper of the insertion tip begins.

Because of this, the placement or introducer catheter of the invention here provides two important properties. First, the tapered tip for insertion has a smooth outer surface with no grooves or any irritating properties to it, which facilitates ready insertion through the patient's skin. Moreover, because the grooves end at the place where the tapered tip begins, there is greater strength and resistance to peelback of the catheter during penetration through the skin.

Before describing this invention in more detail, it may be well to note that the main body of the introducer catheter of the invention is produced in the manner described and claimed in above-noted EP-A-0 279 015. That is, by coextrusion of a base polymer with a differing polymer forming the v-shaped grooves diametrically placed on either side thereof, the main body portion of the introducer catheter is formed with precisely v-shaped grooves diametrically positioned on either side of the longitudinal extent of the body. This procedure has the effect, however, of forming the grooves in the tip, as well. Thus, by taking the preformed introducer catheter as discussed above, and then forming the tapered tip in accordance with this invention, an introducer catheter is provided with a smooth outer surface at the introducer tip portion thereof.

Preferably, the body of the introducer catheter, in accordance herewith is comprised of polyurethane. However, other materials may be selected. As discussed in EP-A-0 279 015, the strip material used for coextrusion to form the main body of the introducer catheter of the invention here may be an olefin such as polyethylene. Polyurethane is preferred as the main body of the introducer catheter, because it is biocompatible and has great strength, permitting a thinner wall and greater kink resistance.

Other objects and advantages of this invention will be apparent from the following description, the accompanying drawings, and the appended claims.

DESCRIPTION OF THE DRAWINGS

Fig. 1 is a cross-sectional view of the main catheter body of the invention showing the co-extruded nature of the formation thereof;

Fig. 2 is a cross-sectional view of the catheter of Fig. 1 with the coextruded strip material removed to show the remaining cross-sectional view of the main introducer catheter body of the invention;

Fig. 3 is a partial perspective view of the introducer catheter of the invention and showing the tapered tip of the distal end; and

Fig. 4 is a sectional view of a die arrangement for forming the catheter of the invention, with a mandril positioned all the way therein to define the space into which the heated catheter body of the invention flows during the formation of the tip of the introducer catheter of the invention.

## DETAILED DESCRIPTION OF THE INVENTION

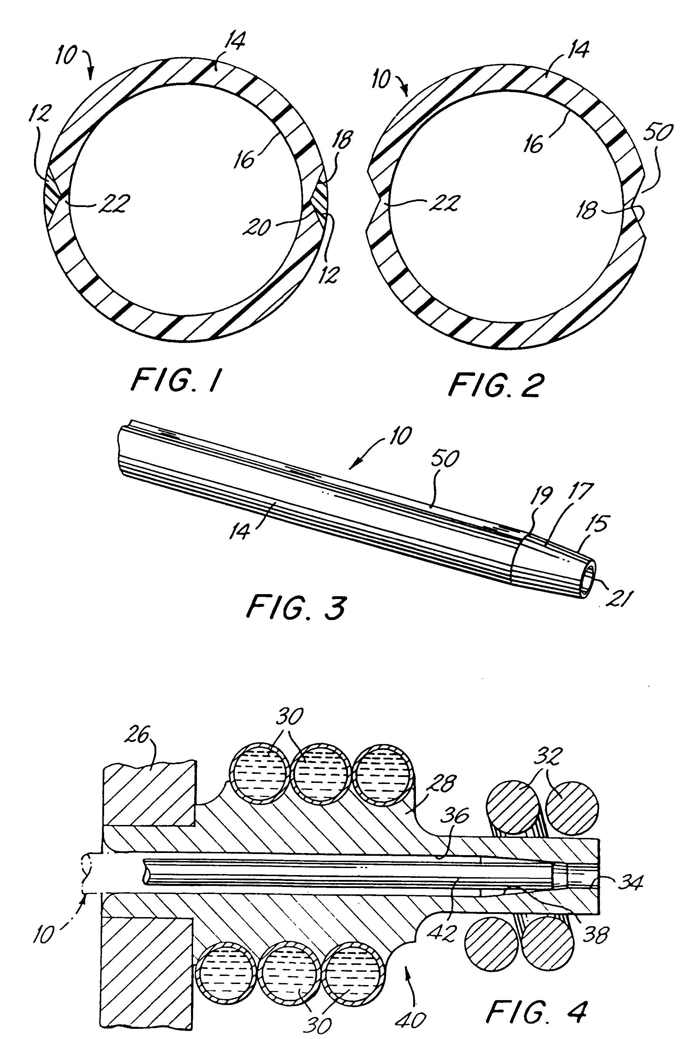

Referring to the drawings in which like reference characters refer to like parts throughout the several views thereof, Fig. 1 shows the cross section of the catheter formation assembly generally designated 10 with a catheter body 14 and the coextruded diametrically positioned strips 12. As discussed above, body 14 may be comprised of polyurethane whereas strips 12 may be comprised of polyethylene. Because of the dissimilar polymeric structure of 12 and 14, the opposed surfaces 18, 20 do not readily adhere to each other so that strips 12 may be readily stripped from body 14 leaving the arrangement, as shown in Fig. 2. As can be seen in both Figs. 1 and 2, the longitudinal extent of body 14 of the introducer catheter of the invention has weakened areas 22 of greatly reduced cross-sectional dimension between inner surface 16 of the lumen and outer surface 23 so that there is a ready stripping and separation of the two halves of the body 14 when desired.

Referring to Fig. 3, the catheter assembly 10 is shown in a perspective view with the tapered tip positioned on the distal end 21 thereof. That is, beginning at point 19, tip 15 has a tapered surface 17 for ease of insertion of the introducer catheter through the skin of a patient. As can be seen further in Fig. 3, one of the longitudinal grooves 12 is shown which facilitates splitting of the introducer assembly from the rest of a catheter apparatus when required. Nevertheless, the groove 12 does not continue or extend beyond point 19. Thus, tapered surface 17 is smooth so that no additional trauma is developed during insertion. Moreover, because no groove is positioned at the distal end 21 or at any portion of the surface of the tip 15, there is no peelback of the two halves of the introducer catheter assembly 10 during penetration of the skin.

Referring now to Fig. 4, a die and mandril assembly 40 is shown for producing the smooth surfaced tapered tip 15 of the introducer catheter of the invention. Die 28 defines an interior surface 36 therein which has a tapered molding portion 38 that is tapered according to the tip configuration desired on the catheter 10 of the invention. It will be understood, in this connection, that dies 28 are interchangeable for producing tips with differing tapers as may be required. Additionally, interior surface 36 has an opening 34 in die 28 near the tapered surface 38. With the exception of tapered surface 38, interior surface 36 defines a cylindrical cavity within die 28.

Initially, prior to the reformation of a tip, in accordance with the method of the invention here, a re-circulating pump is energized to cause cooling liquid to continuously flow through the coils 30 wrapped around die 28.

The catheter body 10 to be tipped is mounted to be even with or to extend slightly beyond mandril 42, as shown in Fig. 4, which is supported in cantilever fashion from a movable carriage, not shown as will be understood and as shown and discussed in more detail in the above-noted US-A-4,661,300. Catheter 10 may be first coated with a lubricating agent, which may be a silicone lubricant, to facilitate its insertion into and removable from die 28.

A radio-frequency generator may be connected to heating coils 32, and is energized to begin the heating of die 28 prior to insertion of the mandril 42, catheter 10 assembly into die 28. The level of heating is to a temperature only sufficiently high that the thermoplastic material forming catheter 10 will just begin to melt and flow when it is inserted into die 28. As will be understood by practitioners-in-the-art, a control is arranged to maintain the desired temperature for causing flow of the polymer, as previously selected, to maintain die 28 at that temperature.

Once die 28 has been heated to the desired temperature, catheter 10 is moved by mandril 42 into contact with molding surface 38 of die 28 by the movement of the mandril 42 into the die 28.

As catheter 10 on mandril 42 moves into die 28 the catheter tip 15 touches the tapered surface 38. This causes an initial melting of catheter 10 and flow of the surface thereof. As the catheter tip 15 flows, distal end 21 and tip surface 17 flow into the space defined between the mandril 42 and the tapered surface 38. This has the effect of forming the tapered tip surface 17 of tip 15. The mandril 42, in its forward movement into die 28, is set to finish the lead edge 21 of catheter 10 with a severing or clipping motion resulting from its final movement into die 28, ending with contact between mandril 42 and tapered surface 38.

The termination of the heating cycle is coordinated with this final movement into die 28, so that the already continuously circulating cooling water in coils 30 take over for cooling die 28 and the formed tip so that when mandril 42 and catheter body 10 are removed by reversing the movement thereof, catheter 10 has a properly formed and tapered tip 15 with no grooves 12 on the outer surface 17.

Thus, the taper of the tip and the smooth surface thereof are achieved by the flow of melted polymer on the surface of the tip in conjunction with the forward movement of the catheter to fill the mold space for the tip with the tapered surface of the die. The degree of flow is controlled by the selection of temperature and cooling in conjunction with the forward movement.

While the method herein described and the resulting introducer catheter so formed constitute preferred embodiments of this invention, it is to be understood that the invention is not limited to this precise method and arrangement of catheter so formed, and changes may be made in either without departing from the scope of the invention, which is defined in the appended claims.

## Claims

1. A splittable introducer catheter (10) comprising
a) an elongated tubular body (14) comprised of a thermoplastic polymer,
b) said body being defined by a wall having a first. radial cross sectional thickness through the length thereof,
c) said wall having an inner surface and an outer surface,
d) the inner surface of said wall defining a lumen extending centrally through the length of said body,
e) a tapered tip 15 positioned on the distal end of said body;
characterised in that two diametrically opposed v-shaped grooves (12) are provided in said outer surface of said body, said grooves extending the length of said body, but ending at the point where the taper of said tip begins so that the outer surface (17) of said tapered tip has a continuous shape with no grooves.

2. A method for producing a catheter according to claim 1 which comprises:
a) mounting a grooved splittable introducer catheter to be tipped coaxially over a support mandril (42) whereby said catheter is even with or extends slightly beyond the end of said mandril,
b) heating a die (28) having an interior molding surface (36) at least one portion of which is tapered according to the tip configuration desired on said splittable catheter, and said surface being open at both ends for allowing flow of catheter material therethrough,
c) guiding said mandril toward said die such that said splittable catheter carrying mandril enters said die and carries said splittable catheter toward engagement with said tapered molding surface,
d) continuing to move said mandril into said die for forcing said splittable catheter into the space established beteen said mandril and said tapered molding surface of said die,
e) allowing said splittable catheter to heat and flow in and through said space between said tapered molding surface of said die and said mandril,
f) advancing said flowing heated splittable catheter and carrying mandril until said mandril engages said tapered molding surface for removing said v-shaped grooves at the forming, flowing tip of said splittable catheter,
g) severing the flowing tip of said splittable catheter where said mandril engages said interior tapered molding surface.
h) cooling said die and said splittable catheter while holding said splittable catheter in said space defined by said mandril and said tapered die surface,
i) reversing movement of said mandril and splittable catheter so that-said splittable catheter is withdrawn from said die; and
j) removing said formed splittable catheter with a smooth surfaced tip.

3. The method of claim 2 wherein the smooth surfaced tip (15) is defined by the engagement of the splittable catheter with the tapered molding surface (36).

4. The method of claim 2 or 3 wherein the temperature of said catheter material is measured during heating and melting for controlling the movement of said splittable catheter into said die.

5. The method of claim 2, 3 or 4 wherein the temperature of said die is measured to determine the cooling of said catheter in the space defined between said mandril and said tapered die molding surface.

## Patentansprüche

1. Spaltbarer Einführkatheter (10) mit
a) einem langgestreckten rohrförmigen Körper (14) aus. einem plastischen Polymer, wobei
b) dieser Körper von einer Wandung mit einer sich über. die Länge des Körpers erstreckenden ersten radialen Querschnittsdicke gebildet ist,
c) diese Wandung eine Innenfläche und eine Außenfläche besitzt,
d) die Innenfläche der Wandung eine sich zentrisch über die Länge des Körpers erstreckende Seele begrenzt und
e) am distalen Ende dieses Körpers eine sich verjüngende Spitze (15) vorgesehen ist,
dadurch gekennzeichnet, daß in der Außenfläche des erwähnten Körpers zwei einander diametral gegenüberliegende V-förmige Nuten (12) vorgesehen sind, welche sich über die Länge des erwähnten Körpers erstrecken, jedoch an einer Stelle enden, wo die Verjüngung der Spitze beginnt, so daß die Außenfläche (17) der sich verjüngenden Spitze eine ununterbrochene Gestalt ohne Nuten besitzt.

2. Verfahren zum Herstellen eines Katheters nach Anspruch 1, bei welchem
a) ein genuteter spaltbarer und mit einer Spitze zu versehender Einführungskatheter coaxial auf einem Stützdorn (42) in solcher Weise montiert wird, daß der Katheter mit dem Ende des Stützdorns eben abschließt oder geringfügig über dessen Ende ragt,

b) eine eine innere Formfläche (36) aufweisende Form (28), deren Formfläche zumindest zum Teil entsprechend der gewünschten Gestalt der Spitze am spaltbaren Katheter verjüngt zuläuft und an beiden Enden offen ist, um Kathetermaterial ausfließen zu lassen, erhitzt wird,

c) der Stützdorn in solcher Weise zur Form hin geführt wird, daß der den spaltbaren Katheter tragende Stützdorn in die Form eintritt und den spaltbaren Katheter bis zur Anlage an der sich verjüngenden Formfläche trägt,

d) der Stützdorn weiterhin in die Form bewegt wird, um den spaltbaren Katheter in den zwischen dem Stützdorn und der sich verjüngenden Formfläche der Form vorhandenen Raum zu drücken,

e) zugelassen wird, daß sich der spaltbare Katheter erhitzt und in und durch den Raum zwischen der sich verjüngenden Formfläche der Form und dem Dorn fließt,

f) der fließende, erhitzte spaltbare Katheter und der Stützdorn vorgeschoben wird, bis der Stützdorn die sich verjüngende Formfläche berührt, um die V-förmigen Nuten an der dem Verformen unterworfenen fließenden Spitze des spaltbaren Katheters zu beseitigen,

g) die fließende Spitze des spaltbaren Katheters dort abgetrennt wird, wo der Stützdorn die innere, sich verjüngende Formfläche berührt,

h) die Form und der spaltbare Katheter gekühlt wird, während der spaltbare Katheter in dem Raum zwischen dem Stützdorn und der sich verjüngenden Formfläche gehalten wird,

i) die Bewegung des Stützdorns und des spaltbaren Katheters umgekehrt wird, so daß der spaltbare Katheter aus der Form herausgezogen wird und

j) der geformte, mit einer Spitze mit glatter Oberfläche versehene spaltbare Katheter entfernt wird.

3. Verfahren nach Anspruch 2, bei welchem die mit glatter Oberfläche versehene Spitze (15) dadurch ausgebildet wird, daß der spaltbare Katheter mit der sich verjüngenden Formfläche (36) in Berührung gebracht wird.

4. Verfahren nach Anspruch 2 oder 3, bei welchem die Temperatur des Kathetermaterials während des Erhitzens und des Schmelzens gemessen wird, um die Bewegung des spaltbaren Katheters in die Form hinein zu steuern.

5. Verfahren nach Anspruch 2, 3 oder 4, bei welchem die Temperatur der Form gemessen wird, um die Abkühlung des Katheters in dem zwischen dem Stützdorn und der sich verjüngenden Formfläche ausgebildeten Raum zu bestimmen.

## Revendications

1. Un cathéter d'introduction pelable (10) comprenant

a) un corps tubulaire allongé (14) constitué d'un polymère thermoplastique,

b) ledit corps étant défini par une paroi ayant une première épaisseur. à section radiale sur toute sa longueur,

c) ladite paroi ayant une surface intérieure et une surface extérieure,

d) la surface intérieure de ladite paroi définissant un espace s'étendant centralement sur toute la longueur du dit corps,

e) une pointe effilée (15), placée à l'extrémité distale du dit corps;

caractérisé en ce que deux rainures en V diamétralement opposées (12) sont aménagées dans ladite surface extérieure du dit corps, lesdites rainures s'étendant sur la longueur du dit corps mais se terminant au point où commence l'effilement de ladite pointe, de sorte que la surface extérieure (17) de ladite pointe effilée a une forme continue, sans rainures.

2. Un procédé de production d'un cathéter selon la revendication 1, comprenant les étapes suivantes:

a) montage d'un cathéter d'introduction pelable à rainures, devant être formé en pointe, coaxialement sur un mandrin de support (42), ledit cathéter étant au même niveau que l'extrémité du dit mandrin ou s'étendant légèrement au-delà de celle-ci,

b) échauffement d'une filière (28), comportant une surface de moulage intérieure (36), dont au moins une partie est effilée en fonction de la configuration de la pointe voulue sur ledit cathéter pelable, ladite surface étant ouverte aux deux extrémités pour permettre l'écoulement du matériau du cathéter,

c) guidage du dit mandrin en direction de ladite filière, ledit mandrin supportant ledit cathéter pelable rentrant dans ladite filière et entraînant ledit cathéter pelable en vue de son contact avec ladite surface de moulage effilée,

d) poursuite de l'avancement du dit mandrin dans ladite filière en vue de l'engagement du dit cathéter pelable dans l'espace formé entre ledit mandrin et ladite surface de moulage effilée de ladite filière,

e) échauffement du dit cathéter pelable et écoulement de celui-ci à travers le dit espace entre ladite surface de moulage effilée de ladite filière et du dit mandrin,

f) avancement du dit cathéter pelable échauffé en écoulement et du dit mandrin de support jusqu'à ce que ledit mandrin entre en contact avec ladite surface de moulage pour supprimer lesdites rainures en V de la pointe formée et en écoulement du dit cathéter pelable,

g) séparation de la pointe en écoulement du dit cathéter pelable au point où ledit mandrin entre en contact avec ladite surface de moulage intérieure effilée

h) refroidissement de ladite filière et du dit cathéter pelable en maintenant ledit cathéter pelable dans ledit espace défini par ledit mandrin et ladite surface de filière effilée,

i) inversion du mouvement du dit mandrin et du dit cathéter pelable, ledit cathéter pelable étant ainsi extrait de ladite filière; et

j) enlèvement du dit cathéter pelable formé avec une pointe à surface lisse.

3. Le procédé selon la revendication 2, dans lequel la pointe à surface lisse (15) est définie par la mise en contact entre le cathéter pelable et la surface de moulage effilée (36).

4. Le procédé selon la revendication 2 ou 3, dans lequel la température du dit matériau du cathéter est mesurée pendant l'échauffement et la fusion en vue du contrôle de l'avancement du dit cathéter pelable dans ladite filière.

5. Le procédé selon la revendication 2, 3 ou 4, dans lequel la température de ladite filière est mesurée pour déterminer le refroidissement du dit cathéter dans l'espace défini entre ledit mandrin et ladite surface de moulage effilée.

FIG. 1

FIG. 2

FIG. 3

FIG. 4